# EUROPEAN PATENT SPECIFICATION

(11) **EP 2 656 072 B1**
(45) Date of publication and mention of the grant of the patent: **21.01.2015**
(21) Application number: 11815444.2
(22) Date of filing: 20.12.2011
(51) Int. Cl.: G01N 33/53, G01N 33/536, G01N 33/543, G01N 33/558

(54) **MULTI-ANALYTE MICROARRAYS USING TAG-SPECIFIC ANTIBODIES AND TAG-ANCHORED ANTIBODIES**
MULTIANALYT-MIKROARRAYS MIT TAG-SPEZIFISCHEN ANTIKÖRPERN UND TAG-ANKERANTIKÖRPERN
MICRORÉSEAUX D'ANALYSATS MULTIPLES UTILISANT DES ANTICORPS SPÉCIFIQUES D'ÉTIQUETTES, ET ANTICORPS ANCRÉS SUR DES ÉTIQUETTES

(30) Priority: 23.12.2010 GB 201021909
(43) Date of publication of application: 30.10.2013
(73) Proprietor: Randox Laboratories Ltd., Crumlin, County Antrim BT29 4QY (GB)
(72) Inventor: BENCHIKH, Elouard, Crumlin Antrim BT29 4QY (GB); FITZGERALD, Peter, Crumlin Antrim BT29 4QY (GB); MCCONNELL, Ivan, Crumlin Antrim BT29 4QY (GB); LOWRY, Philip, Crumlin Antrim BT29 4QY (GB)
(86) International application number: PCT/EP2011/073359
(87) International publication number: WO 2012/084915

(56) References cited:
- WO-A1-93/17335
- US-A1- 2004 049 351

## Description

### BACKGROUND

The multi-analyte microarray (MAMA) is an effective tool for the simultaneous detection and quantification of multiple molecules in various sample matrices. The advantages of the biochip compared to traditional single analyte analysis include reduced sample volume and high-throughput capability. It has important uses in drug development, environmental testing, residue monitoring, veterinarian practice, and especially, clinical chemistry and basic biological research. The first successfully automated commercialisation of the MAMA concept was Randox's biochip technology (for the purposes of the invention the words biochip and microarray are synonymous and are used interchangeably) incorporated on the Evidence^{™} and Evidence Investigator^{™} platforms (EP0874242; EP0902394; EP0988893; EP0994355; EP1227311; EP1273349; Clinical Chemistry 2005, 51(7): 11665-1176). The biochip is a ceramic-coated substrate to which a number of specific binding partners are either covalently attached or passively adsorbed at specific locations. The specific binding partners are molecules capable of binding to target analytes in a sample, often antibodies or fragments thereof. The use of various labeling techniques enables the visualization of the interaction of a specific binding partner and an analyte and calibrators enable analyte quantification. Generally, MAMAs are commercialized as a manufacturer-defined end product i.e. a set of analyte-specific antibodies chosen by the company commercializing developing, manufacturing and selling the product. Currently, the combination of analytes chosen for a MAMA is scientifically coherent, being grouped, for example, by physiological function or association with a specific disease state e.g. Randox Cytokine I supports antibodies to IL-1α, IL-1β, IL-2, I1-4, IL-6, IL-8, IL-10, IFN-γ, TNF-α, MCP-1, EGF and VEGF, proteins recognized by the scientific community as important in inter-cellular signaling during an immune response. However, research scientists by definition are constantly testing new ideas and theories and may require biochips that recognize analyte combinations that are scientifically speculative. Although MAMAs can be developed to order, such development is time-consuming and costly. In the context of the current invention, reference will be made to static and flexible MAMAs. Static implies a format with a manufacturer-defined analyte specificity while flexible implies that the end-user can both define the analyte specificity and acquire it off-the-shelf. The static and flexible protein MAMA concepts were proposed by Ekins (1989; EP0749581). The EP0749581 flexible MAMA is underpinned by the DNA hybridization reaction; various capture agents in the form of short-chain oligonucleotides are tethered to the solid support and bind to their complementary sequenced oligonucleotides which are attached (as 'tails') to specific analyte-binding agents such as antibodies. The flexible MAMA format based on oligonucleotides attached to a solid support as capture agents has disadvantages compared to the format described by the current invention which uses Tag-specific antibodies attached to the solid support as capture agents. Firstly, the temperatures required to gain oligonucleotide specificity in a multi-analyte array (approximately 40-60°C based on oligonucleotides of 18-20 base pairs) would likely have a sample denaturing effect and/or affect the conjugation of antibodies to the oligonucleotides (Breslauer et al. 1986; Rychlik et al. 1990). Secondly, due to cross-hybridisation the oligonucleotide format is less accurate. For example, in EP20040759957, a cross-hybridisation level of no greater than 0.1 % is offered as an assay attribute, yet in the current invention, the cross-reactivity values of the various monoclonal antibodies of the invention have cross-reactivities for non-target tag molecules of less than 0.00001 % (the limit of measurement of the cross-reactivity assay). Thus the MAMA format described by the current invention has no reagent problems related to increased temperatures, reduces the risk of cross-reactivity, and therefore enables a more accurate assessment of the analyte content of a patient sample. There is no mention in EP0749581 of using a solid support supporting Tag-specific antibodies as the capture agent. Although the oligonucleotide tail MAMA was proposed in 1990 the Inventors are not aware of any commercialization of the method. What is required, and which is solved by the current invention, is the provision of off-the-shelf, customisable reagents for flexible MAMA development that retains or betters the performance characteristics of the traditional static MAMA. The invention is underpinned by Tag-specific antibodies attached to a solid support that bind analyte-specific Tag-anchored antibodies. The invention is applicable to traditional competitive and sandwich assay formats, as well as a mixture of the two formats.

### References

Ekins R.P. et al. (1989). The development of microspot, multi-analyte radiometric immunoassay using dual fluorescent-labeled antibodies. Anal Chim Acta, 227:73-96 Fitzgerald S.P. et al. (2005). Clinical Chemistry 51: 1165-1176
Breslauer K.J. et al. (1986). Predicting DNA duplex stability from the base sequence. PNAS, 83: 3746-3750.
Rychlik W. et al. (1990). Optimization of the annealing temperature for DNA amplification in vitro. Nuc. Ac. Res., 18: 6409-6412.

### SUMMARY OF THE INVENTION

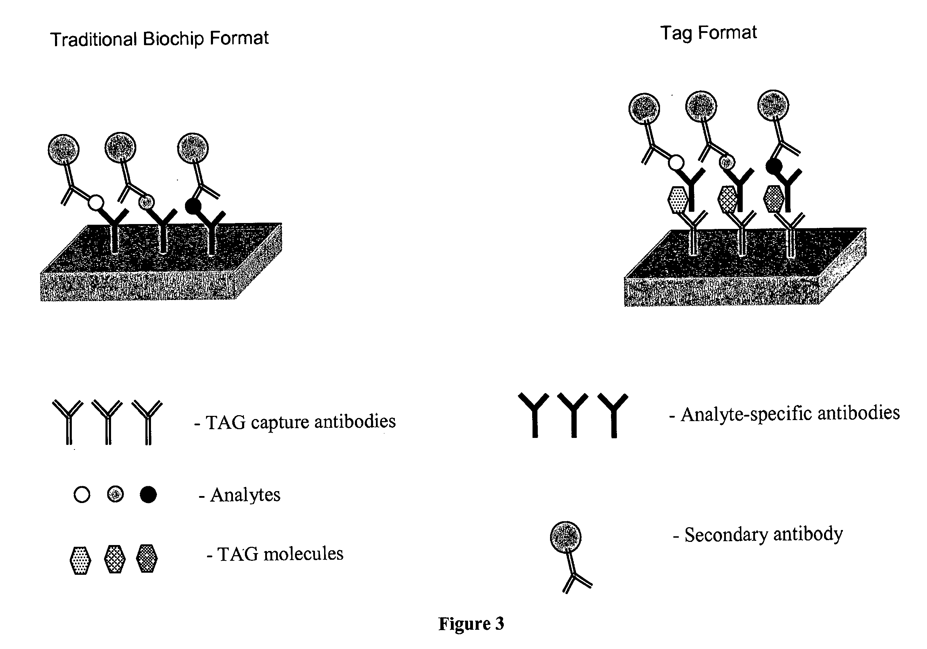

The invention described herein is a method, underpinned by antibody capture agents attached to a solid support and analyte-specific Tag-anchored antibodies that is sensitive, accurate and overcomes the inflexible, expensive nature of current static biochips enabling the end-user to choose and develop personalised MAMAs. Besides empowering the end-user, the system reduces the costs of manufacture leading to a more affordable end-product and is more accurate than the oligonucleotide-based flexible MAMA. This is achieved through the enhancement of the traditional biochip system by using Tag-specific antibodies attached to a solid support and analyte-specific Tag-anchored antibodies (Figure 3). The invention also provides a kit based on the Tag-specific antibodies and analyte-specific Tag-anchored antibodies for detecting and/or determining two or more analytes in a sample.

### DRAWINGS

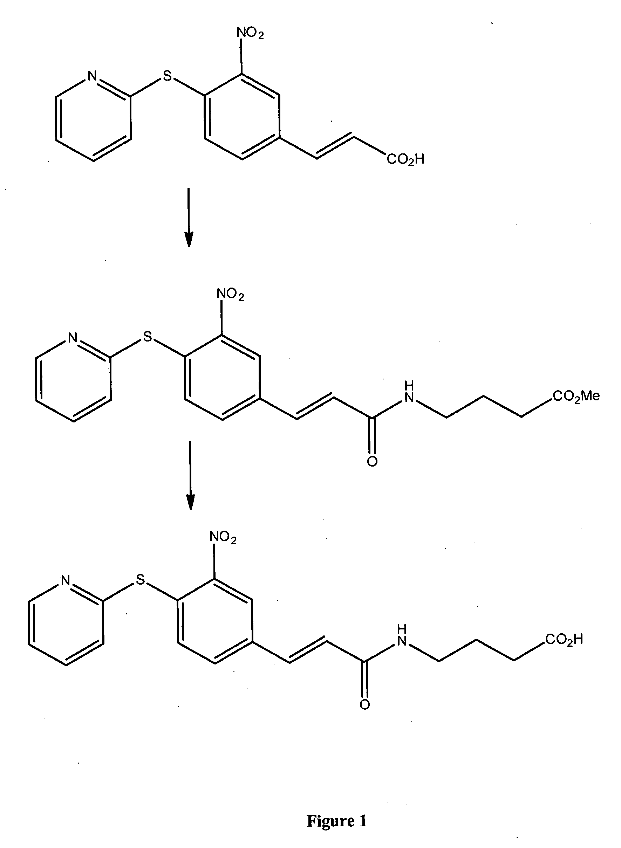

**Figure 1** Synthesis of Tag 9

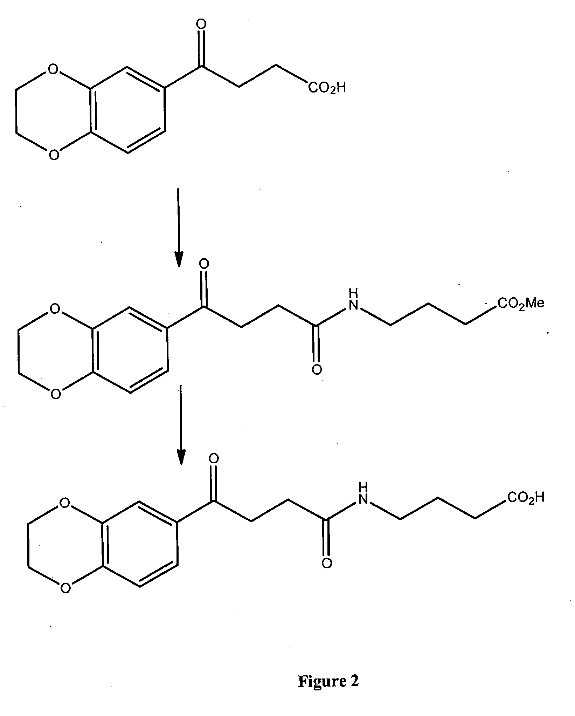

**Figure 2** Synthesis of Tag 11
**Figure 3** Sandwich immunoassay format for traditional biochip and Tag biochip
**Figure 4** Examples of Tag molecules

### DETAILED DESCRIPTION OF INVENTION

In a first aspect, the invention is a method which enables the flexible MAMA for detecting or determining two or more analytes in a sample, the method comprising adding the sample, two or more analyte-specific Tag-anchored antibodies and two or more labeled conjugates to a solid support comprising two or more Tag-specific antibodies at spatially-defined locations on the solid support, each Tag-specific antibody recognizing a different Tag; and detecting or determining the amount of two or more analytes in the sample by detecting and measuring the labeled conjugates and comparing the measurement values with the measurement values from calibrators characterized in that the two or more analyte-specific Tag-anchored antibodies comprise at least one analyte-specific Tag-anchored antibody which is specific for an analyte that is a macromolecule and at least one analyte-specific Tag-anchored antibody which is specific for an analyte that is a small molecule with a molecular weight of less than 5,000 Daltons.

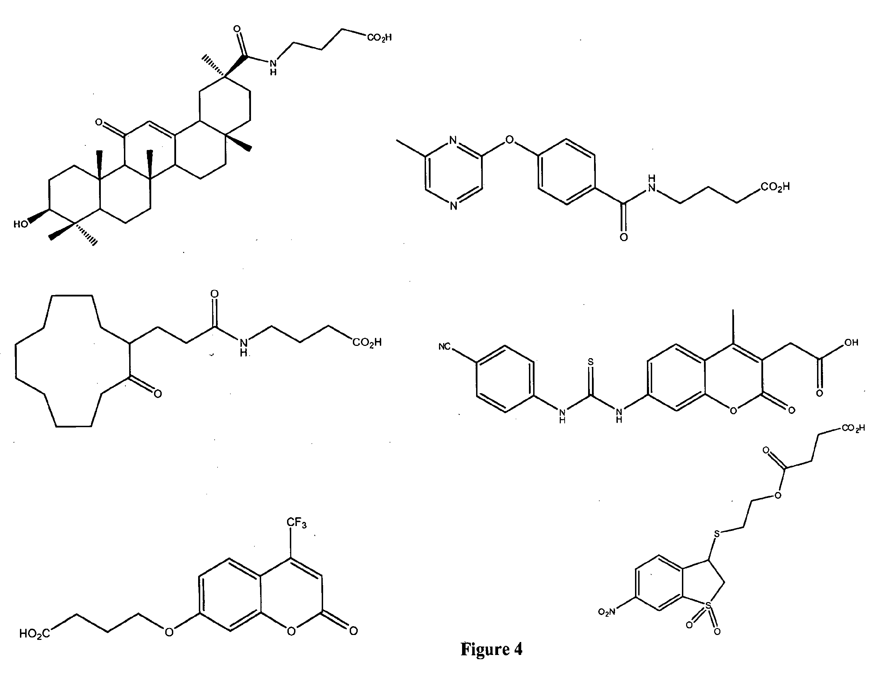

The Tag is a molecule which is, not a biochemical and is structurally distinct from commonly targeted analytes. The Tag can be for example a small molecule, polymer, peptide, polypeptide, oligonucleotide or protein. Preferably the Tag is a small molecule of molecular weight less than approximately 5000 Daltons, preferably less than approximately 1000 Daltons. Exemplary Tag molecules are shown in Figure 4 The Tag-specific antibody is a highly specific polyclonal antibody, a monoclonal antibody, a highly specific antibody fragment such as a scFV, a highly specific antibody hybrid such as a humanized antibody, or any other antibody-based agent. Preferably, the Tag-specific antibody is a monoclonal antibody. For the current invention, the term 'analyte-specific Tag-anchored antibody' can mean that the Tag-anchored antibody either recognizes a single analyte or a well-defined group of analytes possessing a common epitope that is recognised by the Tag-anchored antibody. Detecting means qualitatively analyzing for the presence or absence of an analyte or analytes. This may require, for example, exceeding the well known analytical concept the limit of detection, or alternatively, a predefined cut-off concentration(s) set for the analyte(s); the latter can be referred to as a semi-quantitative method. Determining means quantitatively analyzing the amount(s) of analyte(s). Whether detecting or determining analyte(s), as is standard in the art, controls and/or calibrators are required. The calibrators may be previously measured values or may be values derived concurrently with the analytes to be measured. The previously measured calibrator values may be stored in a database. The sample can be any biological fluid but is preferably, oral fluid, urine, serum, plasma or blood. Environmental and food samples, after the appropriate pre-treatment steps if required, can also be tested using the invention. Environmental samples to be tested could be, for example, water and soil samples for the detection and/or quantification of contaminants. The solid support is a material capable of supporting an antibody either through a chemical reaction between the antibody and the molecules making up the solid support surface or a derivatised antibody in which the chemical reaction is between the group constituting the derivative of the derivatised antibody and the molecules making up the solid support surface. The solid support is preferably a biochip but can be various substrates such as, for example, inorganic metal oxides, that includes but is not limited to glass, ceramic and silicon. Additionally, the solid support can be a polymer such as (but not limited to) polypropylene, polyvinylene difluoride, and polytetrafluoroethylene. Furthermore, the inorganic solid support can be activated by the process of silanation to introduce chemical functionality, such as epoxy, amine, amide, chloride, isocynate, etc. The preferred solid support is a ceramic biochip. The solid support can also be beads such as magnetic and polystyrene beads and nanoparticles. The antibody or antibody derivative may also be supported by the solid support surface through non-bonding means using molecular cohesive forces such as van der waal's forces. The label of the labeled conjugate is a detectable label such as an enzyme, a luminescent substance, a radioactive substance or a mixture thereof. Preferably the label is a peroxidase, most preferably horseradish peroxidase; the conjugate, for a competitive assay, is a labeled small molecule (or 'hapten') which can compete with the analyte to be detected/measured in the patient sample for the Tag-anchored antibody while the conjugate for a sandwich assay is a labeled antibody which binds to an epitope of the target macromolecule e.g. peptide or protein.

The flexible MAMA format, while enabling traditional competitive and sandwich immunoassay formats, also enables a mixture of the two formats. This allows co-analysis of small molecules (usually analysed using a competitive format) and macromolecules such as peptides or proteins (usually analysed using a sandwich format). The invention also includes a method in which the flexible and static MAMA formats are combined; this involves detecting or determining two or more analytes in a sample comprising adding the sample, one or more analyte-specific Tag-anchored antibodies and two or more labeled conjugates to one or more analyte-specific antibodies (static MAMA) and one or more Tag-specific antibodies (flexible MAMA) occupying spatially-defined locations on a solid substrate.

The invention further provides a method which enables the flexible MAMA for determining two or more analytes in a sample, in which the labeled conjugates are labeled antibodies for sandwich assays and/or labeled haptens for competitive assays.

A further aspect of the invention is Tag-specific antibodies and Tag-conjugated analyte-specific antibodies for use in flexible MAMAs, in which both sets of antibodies are monoclonal antibodies.

Also described is a kit for detecting or determining two or more analytes in a sample comprising two or more Tag-specific antibodies at spatially-defined locations on a solid support and two or more Tag-conjugated analyte-specific antibodies characterized in that the two or more analyte-specific Tag-anchored antibodies comprise at least one analyte-specific Tag-anchored antibody which is specific for an analyte that is a macromolecule and at least one analyte-specific Tag-anchored antibody which is specific for an analyte that is a small molecule with a molecular weight of less than 5,000 Daltons. Another aspect of the invention is a kit for detecting or determining two or more analytes in a sample comprising one or more Tag-specific antibodies and one or more analyte-specific antibodies occupying spatially-defined locations on a solid support, and one or more analyte-specific Tag-anchored antibodies. The kits of the invention optionally contain one or more labeled conjugates.

Finally, the invention describes one or more Tag-specific antibodies on a solid support together with one or more analyte-specific Tag-anchored antibodies for use in a MAMA. If there is only one Tag-specific antibody on a solid support and one corresponding analyte-specific Tag-anchored antibody the solid support further supports one or more analyte-specific antibodies, the analyte-specific Tag-anchored antibody and one or more analyte-specific antibodies each recognizing different analytes. The solid support is as previously described, but is preferably a biochip, most preferably a ceramic biochip.

### General Methods

### TAG molecules

Suitable Tag molecules include low molecular weight organic molecules such as dyes, peptides, and protecting groups used in organic synthesis.

### Immunogen preparation by conjugation of Tag molecules to carrier proteins

Most antigens are macromolecules that contain distinct antigenic sites or epitopes that are recognized and interact with the various components of the immune system. However, Tag molecules are low molecular weight compounds that can interact with the products of an immune response but cannot induce an immune response on their own. These Tag molecules (Tags can be classed as *haptens* i.e. pre-immunogenic molecules) can be made immunogenic by coupling them to a suitable carrier protein using conventional methods for conjugation of haptens to proteins. Carriers are, typically, antigens of higher molecular weight that are able to cause an immunological response when administered *in vivo.* The most commonly used carriers are all highly immunogenic, large molecules that are capable of imparting immunogenicity to covalently coupled haptens (Tags). Some of the more useful carriers are proteins (bovine serum albumin, bovine thyroglobulin, keyhole limpet hemocyanin and ovalbumin), but others include liposomes, synthetic or natural polymers and synthetically designed organic molecules. The criteria for a successful carrier molecule are the potential for immunogenicity, the presence of suitable functional groups for conjugation with a hapten (a Tag) and reasonable solubility properties even after derivatisation. The coupling chemistry used to prepare an immunogen from a hapten (Tag) and a carrier protein is an important consideration for the successful production and correct specificity of the resultant antibodies. The choice of cross-linking methodology is governed by the functional groups present on the carrier and the hapten (tag) as well as the orientation of the hapten (Tag) necessary for appropriate presentation to the immune system. An associated concern is the potential for antibody recognition and cross-reactivity toward the cross-linking reagent used to effect the conjugation. The current methods used for conjugation of the hapten (Tag) to carrier molecules include: carbodiimide-mediated hapten-carrier conjugation (EDC Method); NHS ester-mediated hapten-carrier conjugation (NHS Method); maleimide(or haloacetyl)-thiol conjugation; glutaraldehyde-mediated hapten-harrier conjugation (glutaraldehyde method); diazonium conjugation (diazo method); Mannich Condensation (formaldehyde method); homobifunctional cross-linker-mediated hapten-carrier conjugation.

### Tag-specific mAbs

The isolation, characterisation and purification of Tag specific monoclonal antibodies is an important component of a MAMA, although highly specific polyclonal antibodies are equally suitable. The monoclonal or polyclonal antibodies may be mammalian derived from, for example, mice or sheep. Other sources of antibodies are insects, plants or antibody library technologies such as phage or yeast display. Alternatively short chain antibody fragments (scFVs) or hybrid antibodies such as humanised antibodies may be used.

### Coupling Tag to test-specific antibody

Conjugation of Tag molecules to the test-specific antibody is achieved using a range of coupling methods, some of which are described above (see Immunogen preparation by conjugation of Tag molecules to carrier proteins). The Tag molecules are coupled via the Fc portion of the test antibody using, for example, the periodate method.

The invention will now be described by way of examples.

### Examples

### 1. Tag Synthesis

*Preparation of 3-[3-nitro-4-(2-pyridylthio)phenyl] acrylic acid-aminobutyric acid(Tag 9)* 3-[3-nitro-4-(2-pyridylthio)phenyl] acrylic acid (1.02g, 3.38mmol), methyl 4-aminobutyrate hydrochloride (779mg, 1.5eq) and EDC hydrochloride (972mg. 1.5eq) in pyridine (20ml) were stirred at RT O/N. The solvents were removed in vacuo and to the resulting orange residue was added CH₂Cl₂ (50ml) and water (20ml). The organic portion was separated, dried over sodium sulphate, filtered and evaporated to dryness. The crude residue was purified by column chromatography (silica gel: 50% ethyl acetate in hexane) to give the title compound (1.27g, 90%) as a yellow solid.

3-[3-nitro-4-(2-pyridylthio)phenyl] acrylic acid-aminobutyric acid methyl ester (1.27g, 3.05mmol) was dissolved in THF (20ml). Water was added followed by potassium hydroxide, 85% (623mg, 3eq). The mixture was stirred at RT for 4h. Solvents were removed in vacuo and the remaining aqueous solution was adjusted to pH 3. The mixture was extracted with EtOAc/THF (1:1) (3 x 100ml). The organic extracts were combined, dried over sodium sulphate, filtered and evaporated to dryness. The resultant solid was recrystallised from minimum amount of methanol containing ethyl acetate to give 3-[3-nitro-4-(2-pyridylthio)phenyl] acrylic acid-aminobutyric acid methyl ester (840mg, 69%) as a yellow solid.

### Preparation of 4-(2,3-Dihydro-1,4-benzodioxin-6-yl)-4-oxobutanoic acid-aminobutyric acid (Tag 11)

4-(2,3-Dihydro-1,4-benzodioxin-6-yl)-4-oxobutanoic acid (1.02g, 4.32mmol), methyl 4-aminobutyrate hydrochloride (995mg, 1.5eq) and EDC hydrochloride (1.24g. 1.5eq) in pyridine (20ml) were stirred at RT overnight. The solvents were removed in vacuo and to the resulting orange residue was added CH₂Cl₂ (50ml) and water (20ml). The organic portion was separated, dried over sodium sulphate, filtered and evaporated to dryness. The crude residue was purified by column chromatography (silica gel: 50% ethyl acetate in hexane) to give 4-(2,3-Dihydro-1,4-benzodioxin-6-yl)-4-oxobutanoic acid-aminobutyric acid methyl ester (1.28g, 88%) as an orange/brown solid. 4-(2,3-Dihydro-1,4-benzodioxin-6-yl)-4-oxobutanoic acid-aminobutyric acid methyl ester (1.28g, 3.79mmol) was dissolved in THF (20ml). Water was added followed by, water (20ml) and potassium hydroxide, 85% (776mg, 3eq). The mixture was stirred at RT for 4h. Solvents were removed in vacuo and the remaining aqueous was adjusted to pH 3. A precipitate resulted which was collected by filtration with water wash. The solid was dried in a dessicator over phosphorous pentoxide giving 4-(2,3-Dihydro-1,4-benzodioxin-6-yl)-4-oxobutanoic acid-aminobutyric acid (Tag-9)(1.07g, 88%) as an off-white solid.

### 2. Tag Immunogen Synthesis

### Conjugation of TAG 9 to BSA

To a solution of 3-[3-nitro-4-(2-pyridylthio)phenyl] acrylic acid-aminobutyric acid (14.5mg, 0.0375mmol) in DMF (1.0ml) was added N,N-dicyclohexylcarbodiimide (DCC) (8.45mg, 0.041mmol) and N-hydroxysuccinimide (4.7mg, 0.041mmol) and the mixture was stirred at room temperature overnight. The dicyclohexylurea formed was removed by filtration and the solution was added dropwise to a solution of BSA (50mg) in 100mM sodium bicarbonate solution (pH 8.5) (5ml). The mixture was then stirred overnight at 4 C. The solution was then dialysed against 50mM phosphate buffer pH 7.2 (3 changes) for 24 hours at 4oC, and freeze-dried to give the Immunogen. MALDI results showed 20.2 molecules of TAG 9 had been conjugated to one molecule of BSA.

### Conjugation of TAG 9 to BTG

To a solution of 3-[3-nitro-4-(2-pyridylthio)phenyl] acrylic acid-aminobutyric acid (52.3mg, 0.135mmol) in DMF (1.0ml) was added N,N-dicyclohexylcarbodiimide (DCC)

(30.6mg, 0.1485mmol) and N-hydroxysuccinimide (17.1mg, 0.1485mmol) and the mixture was stirred at room temperature overnight. The dicyclohexylurea formed was removed by filtration and the solution was added dropwise to a solution of BTG (150mg) in 100mM sodium bicarbonate solution (pH 8.5) (15ml). The mixture was then stirred overnight at 4 C. The solution was then dialysed against 50mM phosphate buffer pH 7.2 (3 changes) for 24 hours at 4oC, and freeze-dried to give the immunogen.

### Conjugation of TAG 11 to BSA

To a solution of 4-(2,3-Dihydro-1,4-benzodioxin-6-yl)-4-oxobutanoic acid-aminobutyric acid (11.88mg, 0.037mmol) in DMF (1.0ml) was added N,N-dicyclohexylcarbodiimide (DCC) (8.25mg, 0.04mmol) and N-hydroxysuccinimide (4.6mg, 0.04mmol) and the mixture was stirred at room temperature overnight. The dicyclohexylurea formed was removed by filtration and the solution was added dropwise to a solution of BSA (50mg) in 100mM sodium bicarbonate solution (pH 8.5) (5ml). The mixture was then stirred overnight at 4 C. The solution was then dialysed against 50mM phosphate buffer pH 7.2 (3 changes) for 24 hours at 4oC, and freeze-dried to give the Immunogen. MALDI results showed 17.6 molecule of TAG-11 had been conjugated to one molecule of BSA.

### Conjugation of TAG 11 to BTG

To a solution of 4-(2,3-Dihydro-1,4-benzodioxin-6-yl)-4-oxobutanoic acid-aminobutyric acid (43.37mg, 0.135mmol) in DMF (1.0ml) was added N,N-dicyclohexylcarbodiimide (DCC) (30.5mg, 0.1485mmol) and N-hydroxysuccinimide (17mg, 0.1485mmol) and the mixture was stirred at room temperature overnight. The dicyclohexylurea formed was removed by filtration and the solution was added dropwise to a solution of BTG (150mg) in 100mM sodium bicarbonate solution (pH 8.5) (15ml). The mixture was then stirred overnight at 4 C. The solution was then dialysed against 50mM phosphate buffer pH 7.2 (3 changes) for 24 hours at 4oC, and freeze-dried to give the immunogen.

### 3. Antibodies for LSD, norketamine, NGAL and FABP

All antibodies were sourced from Randox Laboratories Ltd

### 4. Tag Monoclonal antibodies Development

### Development, Isodation, Characterisation and purification of Tag Specific Monoclonal Antibodies

A series of structurally different Tag haptens were developed for sheep immunisation, two examples of which are provided in this application (i.e. Tag 9 and Tag 11). Each hapten was conjugated to BTG as a carrier protein, to increase antigen recognition in the immune system of the sheep. Three sheep were given an initial priming dose of the Tag immunogen followed by a series of monthly maintenance doses. During the maintenance dose immunization period, polyclonal serum was collected monthly and antibody titre assessed. Two animals were then selected for nodal extraction. The two sheep were given two final peri-nodal boosts at monthly intervals and the axillary and prescapular nodes were collected after the final nodal boost. Lymphocytes were extracted, fused with a hetero-myeloma cell line to create hybridoma cells that were then cultured. The supernatant was collected and screened using both specificity and affinity ELISA methods. Specific anti-tag monoclonal antibodies were selected by negatively selecting those clones which had a signal for more than one tag. Positive hybridomas were cloned to stability by means of limit diluting and purified antibody was collected and tested for affinity. The table below shows an example of this affinity data for two Tag 11 clones.

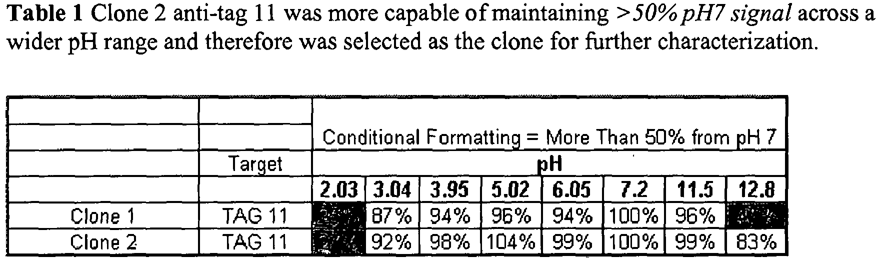

### 5. Tag-Antibody Coupling

### Coupling of Tag 9 to norketamine monoclonal antibody

Norketamine monoclonal antibody (0.5mg) was dialysed against 50mM HEPES solution, pH8.0 with stirring for 2 hours at 15-25°C. 3-[3-Nitro-4-(2-pyridylthio)phenyl]acrylic acid-aminobutyric Acid (Tag 9) (6.0mg) was dissolved in N,N-dimethylformamide (0.6mL). The resulting solution was added to N-hydroxysuccinimide (2.14mg), and pipette up and down until dissolved (this should take no longer than 30 seconds). The resulting solution was added to N-(3-dimethylaminopropyl)-N'-ethylcarbodiimide hydrochloride (3.56mg), and the mixture was incubated on the roller at 15-25°C for 2 hours. N,N-Dimethylformamide (1.4mL) was added to the resulting solution. Conjugation: 4.1µL of the pre-activated Tag 9 was added to antibody to give 10:1 molar ratio of Tag 9:antibody. The resulting solution was incubated on the roller at 15-25°C for 2 hours. Excess hapten was removed with PD-10 column (Pharmacia), pre-equilibrated with PBS (pH 7.2).

### Coupling of Tag 9 to LSD polyclonal antibody

LSD polyclonal antibody (0.5mg) was dialysed against 50mM HEPES solution, pH8.0 with stirring for 2 hours at 15-25°C.

3-[3-Nitro-4-(2-pyridylthio)phenyl]acrylic acid-aminobutyric Acid (TAG 9) (6.0mg) was dissolved in N,N-Dimethylformamide (0.6mL). The resulting solution was added to N-hydroxysuccinimide (2.14mg), and pipette up and down until dissolved (this should take no longer than 30 seconds). The resulting solution was added to N-(3-dimethylaminopropyl)-N'-ethylcarbodiimide hydrochloride (3.56mg), and the mixture was incubated on the roller at 15-25°C for 2 hours. N,N-Dimethylforcnamide (1.4mL) was added to the resulting solution. 4.1µL of the pre-activated TAG 9 was added to LSD polyclonal antibody to give 10:1 molar ratio of TAG 9:antibody. The resulting solution was incubated on the roller at 15-25°C for 2 hours. Excess hapten was removed with PD-10 column (Pharmacia), pre-equilibrated with PBS (pH 7.2).

### Coupling of Tag 11 to LSD polyclonal antibody

LSD polyclonal antibody (0.5mg) was dialysed against 50mM HEPES solution, pH8.0 with stirring for 2 hours at 15-25°C. 4-(2,3-Dihydro-l,4-benzodioxin-6-yl)-4-oxobutanoic acid-aminobutyric acid (TAG 11) (6.0mg) was dissolved in N,N-dimethylformamide (0.6mL). The resulting solution was added to N-hydroxysuccinimide (2.58mg), and pipette up and down until dissolved (this should take no longer than 30 seconds). The resulting solution was added to N-(3-dimethylaminopropyl)-N'-ethylcarbodiimide hydrochloride (4.30mg), and the mixture was incubated on the roller at 15-25°C for 2 hours. N,N-Dimethylformamide (1.4mL) was added to the resulting solution. 3.4µL of the pre-activated TAG 11 was added to antibody to give 10:1 molar ratio of TAG 11 : antibody. The resulting solution was incubated on the roller at 15-25°C for 2 hours. Excess hapten was removed with PD-10 column (Pharmacia), pre-equilibrated with PBS (pH 7.2).

### Coupling of Tag 11 to norketamine monoclonal antibody

Norketamine monoclonal antibody (0.5mg) was dialysed against 50mM HEPES solution, pH8.0 with stirring for 2 hours at 15-25°C. 4-(2,3-Dihydro-1,4-benzodioxin-6-yl)-4-oxobutanoic acid-aminobutyric acid (TAG 11) (6.0mg) was dissolved in N,N-dimethylformamide (0.6mL). The resulting solution was added to N-hydroxysuccinimide (2.58mg), and pipetted up and down until dissolved (this should take no longer than 30 seconds). The resulting solution was added to N-(3-dimethylaminopropyl)-N'-ethylcarbodiimide hydrochloride (4.30mg), and the mixture was incubated on the roller at 15-25°C for 2 hours. N,N-Dimethylformamide (1.4mL) was added to the resulting solution. 3.4µL of the pre-activated TAG 11 was added to antibody to give 10:1 molar ratio of TAG 11:antibody. The resulting solution was incubated on the roller at 15-25°C for 2 hours. Excess hapten was removed with PD-10 column (Pharmacia), pre-equilibrated with PBS (pH 7.2).

### Coupling of Tag 11 to NGAL polyclonal antibody

NGAL polyclonal antibody (0.5mg) was dialysed against 50mM HEPES solution, pH8.0 with stirring for 1 hour at 15-25°C. 4-(2,3-Dihydro-1,4-benzodioxin-6-yl)-4-oxobutanoic acid-aminobutyric acid NHS ester (Tag 11) (5mg) was dissolved in N,N-dimethylformamide (0.5mL). 3.5µL of TAG 11 was added to antibody to give 25:1 molar ratio of TAG 11 :antibody. The resulting solution was incubated on the roller at 15-25°C for 2 hours. Excess hapten was removed with PD-10 column (Pharmacia), pre-equilibrated with PBS containing 0.1 % CAA, 0.01 % MIT (pH 7.2).

### Coupling of Tag 11 to FABP monoclonal antibody

FABP monoclonal antibody (0.5mg) was dialysed against 50mM HEPES solution, pH8.0 with stirring for 1 hour at 15-25°C. 4-(2,3-Dihydro-1,4-benzodioxin-6-yl)-4-oxobutanoic acid-aminobutyric acid NHS ester (Tag 11) (5mg) was dissolved in N,N-dimethylformamide (0.5mL). 3.5µL of TAG 11 was added to antibody to give 25:1 molar ratio of TAG 11 :antibody. The resulting solution was incubated on the roller at 15-25°C for 2 hours. Excess hapten was removed with PD-10 column (Pharmacia), pre-equilibrated with PBS containing 0.1% CAA, 0.01% MIT (pH 7.2).

### 6. MAMA Development and Implementation

Biochip spotting: prior to spotting, biochip surface is silanated (Fitzgerald et al 2005). The Perkin-Elmer BCA spotter was used to spot antibody onto the biochip surface. Monoclonal anti-Tag antibody was spotted (at 10 nL) onto the biochip surface at 1 mg/ml concentration in carbonate buffer (pH - 9.6). Spotted biochips were blocked with 2% Casein in carbonate buffer for one hour at room temperature and then washed with water. Biochips were then stabilised using 5% Trehalose in carbonate buffer for 5 minutes at room temperature and finally dried overnight at 37°C and 25% relative humidity. Biochips were finally chopped and assembled into the carriers and vacuum-sealed for storage at 2-8°C. All immunoassay reagents were sourced from Randox Laboratories Ltd unless otherwise stated. To perform the assay, biochips were loaded with the appropriate volume of assay buffer (100-200 µL), calibrators (25-100 µL) and HRP-labelled conjugate (50-100 µL). Assay buffer was spiked with the appropriate concentration of Tag-conjugated-capture antibody. Biochips were incubated for 30 minutes up to 60 minutes based at 37°C. Finally, biochips were washed using Tris-buffer saline-tween and imaged using 1:1 mixture of luminol/peroxide. For chemi-luminescence imaging luminol/peroxide was mixed 1:1 and 250µl was added in each well. After two minutes of soak in the dark, biochips were imaged using Randox Evidence Investigator.

For NGAL, NKet, LSD assay format: assay diluent (150µl), sample (75µl) incubated at 33°C and 370 rpm for 30 minutes, conjugate (75µl) incubated at 33°C and 370 rpm for 30 minutes.

### Results

**Table 2 Competitive Format - Tag 11/LSD + Tag 9/Norketamine flexible MAMA and single analyte comparisons using biochips**

| | **LSD** | | | **Norketamine** | |
|---|---|---|---|---|---|
| **Concⁿ pg/ml** | **MAMA RLU** | **Single analyte RLU** | **Concⁿ pg/ml** | **MAMA RLU** | **Single analyte RLU** |
| 0.00 | 5622 | 5628 | 0.00 | 10292 | 10033 |
| 30.00 | 5058 | 5167 | 0.98 | 9690 | 9819 |
| 50.00 | 4821 | 5096 | 1.95 | 9357 | 9144 |
| 100.00 | 4079 | 4578 | 3.91 | 7859 | 8200 |
| 156.25 | 3436 | 3677 | 7.81 | 6052 | 6327 |
| 312.50 | 2692 | 3065 | 15.63 | 1753 | 1780 |
| 625.00 | 2230 | 2126 | 31.25 | 1238 | 1362 |
| 1250 | 1324 | 1395 | 62.50 | 594 | 790 |
| 2500 | 1148 | 1036 | 125.00 | 409 | 398 |
| **IC₅₀(pg/ml)** | 287.50 | 396.03 | | 9.39 | 10.10 |

**Table 3 Sandwich and Competitive Format - Tag 11/NGAL + Tag 9/Norketamine flexible MAMA and single analyte comparisons using biochips**

| | **NGAL** | | | **Norketamine** | |
|---|---|---|---|---|---|
| **Concⁿ ng/ml** | **MAMA RLU (B/Bmax)** | **Single analyte RLU (B/Bmax)** | **Concⁿ pg/ml** | **MAMA RLU** | **Single analyte RLU** |
| 0.00 | 58 (1.7) | 62 (1.6) | 0 | 4835 | 5102 |
| 11.40 | 172(5.1) | 184(4.8) | 0.80 | 4342 | 4490 |
| 25.80 | 231 (6.9) | 294 (7.7) | 1.60 | 3976 | 4318 |
| 53.10 | 431 (12.8) | 483 (12.6) | 3.10 | 3803 | 4183 |
| 93.30 | 878(26.1) | 980 (25.6) | 6.20 | 2870 | 2986 |
| 159.20 | 1531(45.5) | 1683(43.9) | 12.50 | 728 | 743 |
| 395.80 | 2459(73.1) | 2526 (65.9) | 25.00 | 429 | 382 |
| 751.40 | 3278 (97.5) | 3282 (85.6) | 50.00 | 229 | 196 |
| 983.20 | 3362 | 3833 | 100.00 | 165 | 131 |
| **IC₅₀ (pg/ml)** | NA | NA | | 7.51 | 7.40 |

**Table 4 Sandwich and Competitive Format - Tag 11/FABP + Tag 9/Norketamine flexible MAMA and single analyte comparisons using biochips**

| | **FABP** | | **Norketamine** | | |
|---|---|---|---|---|---|
| **Concⁿ ng/ml** | **MAMA RLU (%B/Bmax)** | **Single analyte RLU (%B/Bmax)** | **Concⁿ pg/ml** | **MAMA RLU** | **Single analyte RLU** |
| 0.00 | 51(0.2) | 0 | 0 | 4835 | 5102 |
| 2.30 | 363(1.6) | 320(1.4) | 0.80 | 4342 | 4490 |
| 4.90 | 1022 (4.4) | 970(4.1) | 1.60 | 3976 | 4318 |
| 9.00 | 2533(10.8) | 2666(11.3) | 3.10 | 3803 | 4183 |
| 18.70 | 6336(27.1) | 6478(27.5) | 6.20 | 2870 | 2986 |
| 38.40 | 13250(56.7) | 13655(58.0) | 12.50 | 728 | 743 |
| 58.20 | 16933(72.5) | 17634(74.9) | 25.00 | 429 | 382 |
| 73.70 | 20742(88.8) | 20765(88.2) | 50.00 | 229 | 196 |
| 97.40 | 23359 | 23556 | 100.00 | 165 | 131 |
| **IC₅₀ (pg/ml)** | NA | NA | | 7.51 | 7.40 |

The results show that there is no loss in sensitivity in going from the biochip single analyte to the biochip Tag-based flexible MAMA. Furthermore, the sandwich and competitive assay formats can be effectively combined using the Tag concept. The use of Tag-specific antibodies bound to a solid support and analyte-specific Tag-anchored antibodies in effecting flexible MAMAs is thus proven.

## Claims

1. A method for detecting or determining two or more analytes in a sample comprising adding the sample, two or more analyte-specific Tag-anchored antibodies and two or more labeled conjugates to a solid support comprising two or more Tag-specific antibodies at spatially-defined locations on the solid support, each Tag-specific antibody recognizing a different Tag; and detecting or determining the amount of two or more analytes in the sample by detecting and measuring the signal from the labeled conjugates and comparing the measured values with values from calibrators **characterized in that** the two or more analyte-specific Tag-anchored antibodies comprise at least one analyte-specific Tag-anchored antibody which is specific for an analyte that is a macromolecule and at least one analyte-specific Tag-anchored antibody which is specific for an analyte that is a small molecule with a molecular weight of less than 5,000 Daltons.

2. The method of Claim 1 in which the Tags of the analyte-specific Tag-anchored antibodies are small molecules having a molecular weight of less than approximately 5,000 Daltons, preferably less than approximately 1,000 Daltons.

3. The method of the preceding claims in which the labeled conjugates are labeled antibodies for sandwich assays and labeled haptens for competitive assays.

4. A kit for detecting or determining two or more analytes in a sample comprising two or more Tag-specific antibodies at spatially-defined locations on a solid support and two or more tag-conjugated analyte-specific antibodies, **characterized in that** the two or more analyte-specific Tag-anchored antibodies comprise at least one analyte-specific Tag-anchored antibody which is specific for a macromolecule and at least one analyte-specific Tag-anchored antibody which is specific for a small molecule with a molecular weight of less than 5,000-Daltons.

5. The kit of Claim 4 in which the solid support also supports analyte-specific antibodies.

6. Any of the preceding claims in which the solid support is a biochip.

7. Any of the preceding claims in which the sample is a biological fluid, food-derived or derived from the environment.

8. Use of one or more Tag-specific antibodies on a solid support and two or more analyte-specific Tag-anchored antibodies comprising at least one analyte-specific Tag-anchored antibody which is specific for a macromolecule and at least one analyte-specific Tag-anchored antibody which is specific for a small molecule with a molecular weight of less than 5,000 Daltons in a multi-analyte microarray.

## Patentansprüche

1. Verfahren zum Nachweis oder zur Bestimmung von zwei oder mehreren Analyten in einer Probe, umfassend Zugabe der Probe, von zwei oder mehreren Analyt-spezifischen Tag-verankerten Antikörpern und von zwei oder mehreren Konjugaten zu einem festen Träger, der zwei oder mehrere Tag-spezifische Antikörper an räumlich definierten Positionen auf dem festen Träger umfasst, wobei jeder Tag-spezifische Antikörper einen anderen Tag erkennt; und Nachweisen oder Bestimmen der Menge von zwei oder mehreren Analyten in der Probe durch Nachweisen und Messen des von den markierten Konjugaten kommenden Signals und Vergleichen der gemessenen Werte mit Werten von Kalibratoren, **dadurch gekennzeichnet, dass** die zwei oder mehr Analyt-spezifischen Tag-verankerten Antikörper mindestens einen Analyt-spezifischen Tag-verankerten Antikörper, welcher spezifisch für einen Analyten ist, bei dem es sich um ein Makromolekül handelt, und mindestens einen Analyt-spezifischen Tag-verankerten Antikörper, welcher spezifisch für einen Analyten ist, bei welchem es sich um ein kleines Molekül handelt, das ein Molekulargewicht von weniger als 5.000 Dalton aufweist, umfassen.

2. Verfahren nach Anspruch 1, in welchem die Tags der Analyt-spezifischen Tag-verankerten Antikörper kleine Moleküle sind, die ein Molekulargewicht von weniger als ungefähr 5.000 Dalton, vorzugsweise von weniger als ungefähr 1.000 Dalton, aufweisen.

3. Verfahren nach den vorhergehenden Ansprüchen, in welchem die markierten Konjugate markierte Antikörper für Sandwich-Assays und markierte Haptene für kompetitive Assays sind.

4. Kit zum Nachweis oder zur Bestimmung von zwei oder mehreren Analyten in einer Probe, umfassend zwei oder mehrere Tag-spezifische Antikörper an räumlich definierten Positionen auf einem festen Träger und zwei oder mehr Tag-konjugierte Analyt-spezifische Antikörper, **dadurch gekennzeichnet, dass** zwei oder mehr Analyt-spezifische Tagverankerte Antikörper mindestens einen Analyt-spezifischen Tag-verankerten Antikörper, welcher spezifisch für ein Makromolekül ist, und mindestens einen Analyt-spezifischen Tag-verankerten Antikörper, welcher spezifisch für ein kleines Molekül ist, das ein Molekulargewicht von weniger als 5.000 Dalton aufweist, umfassen.

5. Kit nach Anspruch 4, in welchem der feste Träger auch Analyt-spezifische Antikörper unterstützt.

6. Jeder der vorhergehenden Ansprüche, in welchem der feste Träger ein Biochip ist.

7. Jeder der vorhergehenden Ansprüche, in welchem die Probe eine biologische Flüssigkeit ist, die aus der Nahrung oder aus der Umwelt stammt.

8. Verwendung von einem oder mehreren Tag-spezifischen Antikörpern auf einem festen Träger und von zwei oder mehreren Analyt-spezifischen Tag-verankerten Antikörpern, umfassend mindestens einen Analyt-spezifischen Tag-verankerten Antikörper, welcher spezifisch für ein Makromolekül ist, und mindestens einen Analyt-spezifischen Tag-verankerten Antikörper, der spezifisch für ein kleines Molekül ist, das ein Molekulargewicht von weniger als 5.000 Dalton in einem Multianalyt-Microarray aufweist.

## Revendications

1. Procédé de détection ou de détermination d'au moins deux analytes dans un échantillon, comprenant les étapes consistant à ajouter l'échantillon, au moins deux anticorps ancrés sur étiquette spécifiques d'analyte et au moins deux conjugués marqués à un support solide comprenant au moins deux anticorps spécifiques d'étiquette à des emplacements spatialement définis sur le support solide, chaque anticorps spécifique d'étiquette reconnaissant une étiquette différente ; et détecter ou déterminer la quantité d'au moins deux analytes dans l'échantillon en détectant et en mesurant le signal des conjugués marqués et en comparant les valeurs mesurées aux valeurs des étalonneurs, **caractérisé en ce qu'**au moins deux anticorps ancrés sur étiquette spécifiques d'analyte comprennent au moins un anticorps ancré sur étiquette spécifique d'analyte qui est spécifique pour un analyte qui est une macromolécule et au moins un anticorps ancré sur étiquette spécifique d'analyte qui est spécifique pour un analyte qui est une petite molécule d'un poids moléculaire inférieur à 5 000 Daltons.

2. Procédé selon la revendication 1, dans lequel les marqueurs des anticorps ancrés sur étiquette spécifiques d'analyte sont de petites molécules d'un poids moléculaire inférieur à environ 5 000 Daltons, de préférence inférieur à environ 1 000 Daltons.

3. Procédé selon les revendications précédentes, dans lequel les conjugués marqués sont des anticorps marqués pour des dosages sandwich et des haptènes marqués pour les dosages compétitifs.

4. Kit de détection ou de détermination d'au moins deux analytes dans un échantillon comprenant au moins deux anticorps spécifiques d'étiquette à des emplacements spatialement définis sur un support solide et au moins deux anticorps spécifiques d'analyte conjugués à l'étiquette, **caractérisé en ce qu'**au moins deux anticorps ancrés sur étiquette spécifiques d'analyte comprennent au moins un anticorps ancré sur étiquette spécifique d'analyte qui est spécifique pour une macromolécule et au moins un anticorps ancré sur étiquette spécifique d'analyte qui est spécifique pour une petite molécule d'un poids moléculaire inférieur à 5 000 Daltons.

5. Kit selon la revendication 4, dans lequel le support solide supporte également des anticorps spécifiques d'analyte.

6. L'une quelconque des revendications précédentes dans laquelle le support solide est une biopuce.

7. L'une quelconque des revendications précédentes dans laquelle l'échantillon est un fluide biologique, dérivé d'un aliment ou dérivé de l'environnement.

8. Utilisation d'un ou de plusieurs anticorps spécifiques d'étiquette sur un support solide et d'au moins deux anticorps ancrés sur étiquette spécifiques d'analyte comprenant au moins un anticorps ancré sur étiquette spécifique d'analyte qui est spécifique pour une macromolécule et au moins un anticorps ancré sur étiquette spécifique d'analyte qui est spécifique pour une petite molécule d'un poids moléculaire inférieur à 5 000 Daltons dans une micromatrice de plusieurs analytes.
